# EUROPEAN PATENT SPECIFICATION

(11) **EP 4 304 397 B1**
(45) Date of publication and mention of the grant of the patent: **15.10.2025**
(21) Application number: 22711069.9
(22) Date of filing: 08.03.2022
(51) Int. Cl.: A24F 40/05, A24F 40/50, A24F 40/48, B05B 17/06, A24F 40/10, A61M 15/06

(54) **AEROSOL-GENERATING DEVICE USING VIBRATING TRANSDUCER AND CONTROLLED LIQUID SUPPLY**
AEROSOLERZEUGENDE VORRICHTUNG MIT SCHWINGUNGSWANDLER UND KONTROLLIERTER FLÜSSIGKEITSZUFUHR
DISPOSITIF DE GÉNÉRATION D'AÉROSOL UTILISANT UN TRANSDUCTEUR VIBRANT ET UNE ALIMENTATION EN LIQUIDE CONTRÔLÉE

(30) Priority: 10.03.2021 EP 21161710
(43) Date of publication of application: 17.01.2024
(73) Proprietor: Philip Morris Products S.A., 2000 Neuchâtel (CH)
(72) Inventor: DITTMAN, Leander, 2000 Neuchâtel (CH); EMMETT, Robert William, London Greater London EC4M 9AF (GB)
(74) Representative: Reddie & Grose LLP
(86) International application number: PCT/EP2022/055931
(87) International publication number: WO 2022/189450

(56) References cited:
- EP-A1- 3 685 689
- EP-A1- 3 777 583
- WO-A1-2019/239217
- US-A1- 2019 307 170
- US-A1- 2020 367 553

## Description

The present disclosure relates to aerosol-generating devices that use a liquid aerosol-forming substrate. In particular, the disclosure relates to aerosol-generating devices that use a vibrating transducer to atomise liquid aerosol-forming substrate to form an aerosol.

One example of an aerosol generating device is an e-cigarette. Typically, in an e-cigarette a liquid aerosol-forming substrate is heated to generate a vapour that cools to form an aerosol. However, alternative designs have been proposed that use a vibrating transducer to generate droplets from a liquid aerosol-generating device, with the droplets forming an aerosol.

US 2020/367553 A1 relates to an aerosol delivery device comprising an atomization assembly, a reservoir and a control component. The atomization assembly comprises a vibrating assembly that includes a mesh plate. The atomization assembly further includes a pump assembly configured to transfer a portion of a liquid composition from the reservoir to the mesh plate. Delivery of the liquid composition to the mesh plate occurs on demand via control from the control component.

EP 3 685 689 A1 relates to an electronic cigarette liquid supply device including a first interdigital transducer, a second interdigital transducer, a liquid storage chamber, a flow channel and a liquid outlet. A traveling surface acoustic wave excited by the first interdigital transducer actively pumps e-liquid stored in the liquid storage chamber into the flow channel. The traveling surface acoustic wave excited by the second interdigital transducer propagates toward both ends of the flow channel, respectively, to prevent the e-liquid in the liquid storage chamber from flowing into the flow channel and pump the e-liquid in the liquid storage chamber to the liquid outlet

EP 3 777 583 A1 relates to an inhaler comprising a first liquid storage unit; a second liquid storage unit; an atomizing unit which comprises a piezoelectric element substrate having an IDT constructed by use of a pair of interlocking comb-shaped metallic electrodes and is constructed to atomize liquid by a surface acoustic wave generated by applying a high-frequency voltage to the pair of interlocking comb-shaped metallic electrodes; and a mouthpiece for guiding aerosol which is generated by atomizing the liquid in the atomizing unit.

It would be desirable to optimise, as far as possible, the efficiency of any vibrating transducers used in an aerosol-generating device. This is particularly important in handheld aerosol-generating devices, such as e-cigarettes, which are typically battery powered, and are desirably as small as possible, but which need to generate a significant and variable volume of aerosol on demand by a user. There is a need both to maximise battery life by ensuring efficient operation and to allow for variable operating parameters to produce aerosol at different rates.

One particular problem is ensuring efficient operation over a range of different rates of aerosol generation. The vibrating element may become over loaded or under supplied with liquid, leading to inadequate generation of aerosol.

It would be desirable to improve the operation of aerosol-generating devices that use vibrating transducers to provide a range of different rates of aerosol generation and over a range of different operating conditions.

The present disclosure relates to an aerosol-generating device. The aerosol-generating device may comprise a reservoir containing a liquid aerosol-forming substrate. The aerosol-generating device may comprise an aerosol-generating element configured to generate an aerosol from the liquid aerosol-forming substrate. The aerosol-generating element may comprise an electrically operated, vibrating transducer. The aerosol-generating element may comprise an electrically operated pump configured to deliver liquid aerosol-forming substrate from the reservoir to the aerosol-generating element. The aerosol-generating element may comprise control circuitry connected to the pump and the transducer, the control circuitry configured to control the operation of the pump in dependence on one or more operating parameters of the transducer.

According to an aspect of the disclosure, there is provided an aerosol-generating device comprising a reservoir containing a liquid aerosol-forming substrate and an aerosol-generating element configured to generate an aerosol from the liquid aerosol-forming substrate. The aerosol-generating element comprises an electrically operated, vibrating transducer, an electrically operated pump configured to deliver liquid aerosol-forming substrate from the reservoir to the aerosol-generating element, and control circuitry connected to the pump and the transducer. The control circuitry is configured to control the operation of the pump in dependence on one or more operating parameters of the transducer.

The aerosol-generating device may be configured to match the operation of the pump to the operating parameters of the transducer. So, if the transducer is operating at a higher frequency or greater power, the pump can be controlled to deliver more liquid to the pump. This may prevent dry conditions at the transducer. Similarly, if the transducer operates at a lower frequency or lower power level, the pump may be controlled to deliver less liquid. This may prevent or reduce overloading of the transducer with liquid. The control circuitry may be configured to control a rate of delivery of liquid from the reservoir to the aerosol-generating element in dependence on one or more operating parameters of the transducer.

The control circuitry may be configured to drive the transducer. The control circuitry may provide a drive signal to a transducer drive circuit connected to the transducer. The drive signal may determine the operating parameters of the pump.

The one or more operating parameters of the transducer may comprise one or more of: a drive voltage, a drive current, an impedance, a phase difference, a drive frequency a rate of change of impedance, a rate of change of current and a rate of change of voltage.

The operating parameters of the transducer may be determined, at least in part, by a user input to the device. For example, the aerosol-generating device may be designed to generate an aerosol for user inhalation. The device may have a mouthpiece on which a user can puff to draw aerosol out of the device. In this example, the user input may be the strength of a user puff, detected by a flow sensor connected to the control circuitry. A user input may alternatively or additionally be provided through a user interface, such as a button or buttons, or a touch screen. The user may select through the user interface how the transducer operates. This allows the user to customise their experience.

Advantageously, the control circuitry may be configured to receive a feedback signal from the transducer. The feedback signal may comprise one or more operating parameters of the transducer. The control circuitry may be configured to control the operation of the pump in dependence on the feedback signal. The use of a feedback signal from the transducer allows for the operation of the pump to be controlled in reaction to conditions at the transducer. In particular, an oversupply or undersupply of liquid can be determined from the feedback signal or from changes in the feedback signal.

The feedback signal may comprise one or more of: a resonant frequency, an impedance, and a phase. For example, monitoring a phase of the response of the transducer can be advantageous as it is independent of the magnitude of the power. The feedback signal may comprise an amplitude or a rate of change of impedance, current or phase.

The control circuitry is advantageously configured to periodically control the operation of the pump in dependence on the feedback signal. Frequent adjustment of the operation of the pump based on a periodic or continuous monitoring of feedback from the transducer is advantageous to prevent long periods of inefficient or undesirable operating conditions.

The control circuitry may be configured to compare the feedback signal with one or more thresholds to provide a comparison result and to alter operation of the pump in dependence on the comparison result. The control circuitry may be configured to alter the operation of the pump if the feedback signal is not a desired value or within a desired range of values.

The one or more thresholds, the desired value or desired range of values, may be fixed and stored in a memory. Alternatively, the one or more thresholds, the desired value or desired range of values may be calculated based on at least one of: one or more user or sensor inputs, previous values of the feedback signal, and values or expressions stored memory. For example, the control circuitry may monitor changes in the value of the feedback signal over several cycles and alter operation of the pump based on the value of the changes. In another example, fixed thresholds stored in memory and a comparison of the feedback signal with one or more thresholds may be used as the basis for altering operation of the pump.

The control circuitry may also be configured to control the transducer based on the feedback signal. It may be beneficial to control the operating frequency of the transducer or the operating power of the transducer (or both the operating frequency and the operating power of the transducer) in response to changes in the feedback signal from the transducer. Controlling the operating frequency may be beneficial in order to improve the efficiency of the device. Controlling the operating frequency may allow generation of aerosol to be maximised.

The aerosol-generating element may be controlled by the control circuitry to operate in different modes of vibration. Different modes of vibration may give rise to different aerosol volume or different aerosol properties, or both.

The transducer may comprise one or more piezoelectric elements. Piezoelectric elements are preferred because they are an energy-efficient and light-weight means of providing a vibratory output from an electric input. Piezoelectric elements possess a high energy conversion efficiency from electric to acoustic/mechanical power. Further, piezoelectric elements are available in a wide variety of materials and shapes. Inputting an electrical driving signal to a piezoelectric element results in a mechanical output in the form of a vibration. However, as an alternative to the use of a piezoelectric element, actuators consisting of one or more of electromagnetic elements, magnetostrictive elements, or electrostrictive elements may also be employed in the transducer.

The transducer may be part of a transducer assembly that forms the aerosol generating element. The transducer assembly may take different forms. In some embodiments, the transducer assembly comprises a perforated plate, membrane or mesh coupled to the transducer. The transducer may be configured to drive the perforated plate, membrane or mesh into vibration at one or more frequencies. The vibration of the perforated plate, membrane or mesh may force liquid aerosol-forming substrate through the perforated plate, membrane or mesh, which may result in the formation of an aerosol comprising droplets of the liquid aerosol-forming substrate.

The transducer may be configured to drive a plate, membrane or surface into vibration at one or more frequencies. The vibration of the plate, membrane or surface may force the liquid through an adjacent mesh or perforated plate, which may result in the formation of an aerosol comprising droplets of the liquid aerosol-forming substrate. The control circuitry may be configured to drive the transducer in the ultrasonic frequency range, above 20kHz. Preferably, the control circuitry is configured to drive the transducer at a frequency between 50kHz and 300kHz, and more preferably between 100kHz and 200kHz.

The perforated plate, membrane or mesh may comprise one or more nozzles. As used herein, the term "nozzle" refers to an aperture, hole or bore through the plate, membrane or mesh that provides a passage for liquid aerosol-forming substrate to move through the plate, membrane or mesh. The nozzles may be individually sized and arranged relative to each other so as to provide a predetermined desired aerosol droplet formation pattern.

A perforated plate or membrane may be formed of any suitable material. By way of example and without limitation, the plate or membrane may be formed of a polymer material, thereby providing advantages of reduced mass and inertia. However, the plate or membrane may be formed of any other material, such as a metallic material. The plate or membrane may be a composite of two or more different materials. The choice of material(s) used for the plate or membrane may be influenced by the particular liquid aerosol-forming substrate(s) intended to be used with and aerosolised. For example, it is highly desirable to choose a material for the plate or membrane which does not chemically react with or degrade as a consequence of contact with the chosen liquid aerosol-forming substrate. By way of example only, the plate or membrane may be formed of any of palladium, stainless steel, copper-nickel alloy, polyimide, polyamide, silicon or aluminium nitride.

The liquid throughput of the aerosol-generating element depends on various design and operation parameters. For a vibrating perforated plate the throughput is a function of number of nozzles and their diameters. A typical desired throughput for the device may be in the range of 0 µl/sec to 10 µl/sec. This can be achieved, for example, with a perforated plate containing about 800 holes of 5 µm diameter operated at a frequency of about 140 kHz with a drive voltage of 50 V peak-to-peak.

In some embodiments, the transducer assembly comprises an atomising surface configured to contact a liquid aerosol-forming substrate and electrodes on the transducer configured to generate surface acoustic waves (SAW) on the atomising surface. The SAW generate droplets of the liquid aerosol-forming substrate which form an aerosol.

The aerosol-generating device may further comprise a sensor connected to the control circuitry. The control circuitry may be configured to control the operating parameters of the transducer or the pump dependent on an output from one or more sensors.

The transducer may be controlled to operate in response to user demand for aerosol.

The one or more sensors may comprise an airflow sensor. The control circuitry may be configured to operate the pump in response to an input from the airflow sensor. In the example of an aerosol-generating device configured to generate an aerosol for user inhalation, the airflow sensor may detect airflow through the device indicative of a user puff. The control circuitry may activate the pump and the transducer in response to a detected user puff. The aerosol-generating device may comprise a mouthpiece through which a user can draw generated aerosol.

The control circuitry may operate the pump according to user demand based on detected user puffs. The transducer may be operated in a pulsed mode, with a pulse duration of, for example, 1 sec to 3 sec, or as long as the airflow sensor detects an airflow rate above a threshold. The control circuitry may drive the transducer with a voltage, frequency or power that increases with increasing airflow rate to provide more aerosol when the airflow rate is higher.

The one or more sensors may comprises a temperature sensor or a humidity sensor. Changes in ambient temperature or humidity may affect the operation of the transducer and the properties of the generated aerosol. The transducer assembly may also increase in temperature during use, which may alter the resonant behaviour of the transducer and may alter the properties of the liquid aerosol-generating substrate.

The one or more sensors may comprise a sensor for determining an identity of the liquid aerosol-forming substrate. It may be desirable to operate the system with different parameters for different liquid aerosol-forming substrates.

Advantageously, the control circuitry may be configured to stop operation of the pump a predetermined time before stopping operation of the transducer. This reduces the possibility of liquid that has been dispensed from the pump not being converted into aerosol and remaining at the transducer assembly. Such remaining liquid might undesirably leak from the device.

The aerosol-generating device may comprise a user interface, such as a button or buttons, or a touch screen. The control circuitry may be configured to control the operating parameters of the transducer dependent on an input signal from the user interface. This may allow for the user to customise their experience.

The aerosol-generating device may comprise a memory. The control circuitry may be configured to control the operating parameters of the transducer dependent on user preferences stored in memory.

The pump may be a micro pump. As used herein, a micro pump is a pump with dimensions small enough to allow for integration into a handheld device. The micro pump may have functional dimensions in the micrometer range. The micro pump may provide a liquid throughput of a few microliters per second. For example, the micro pump maybe operated to provide a liquid throughput of between 0 µl/sec and 100 µl/sec, between 0 µl/sec and 50 µl/sec or between 0 µl/sec and 10 µl/sec. The pump may be a mechanical pump, a diaphragm pump, a piezoelectric pump, a peristaltic pump, an electro-hydrodynamic or magneto-hydrodynamic pump, an electro-osmotic pump, an adhesion pump, or rotary pump.

The pump may function as a valve, preventing the escape of liquid to the aerosol generating element when not activated. This reduces the possibility for undesirable escape of liquid aerosol-forming substrate from the device.

The aerosol-generating device may be a portable or handheld device. The aerosol-generating device may produce an aerosol for user inhalation. The aerosol-generating device may comprise a mouthpiece, through which a user may draw aerosol generated by the device.

The aerosol-generating device may comprise a power source that supplies power to the pump and to the transducer. The power source may be any suitable power source, for example a DC voltage source such as a battery. Preferably, the power source is rechargeable. In one embodiment, the power supply is a Lithium-ion battery. Alternatively, the power supply may be a Nickel-metal hydride battery, a Nickel cadmium battery, or a Lithium based battery, for example a Lithium-Cobalt, a Lithium-Iron-Phosphate, Lithium Titanate or a Lithium-Polymer battery.

The control circuitry may comprise one or more microprocessors. The one or more microprocessors may comprise a programmable microprocessor. The term control circuitry encompasses any control electronics and processor(s) configured for use in generating the driving signal for the transducer, as well as any computer-readable medium storing instructions for use in the generating of the driving signal. By way of example, the control circuitry may take the form of control electronics and a non-transitory computer readable medium (such as a computer memory module), in which the control electronics comprise a control unit coupled to or containing the non-transitory computer readable medium. The control unit may itself contain or be coupled to a computer processor. The non-transitory computer readable medium may contain instructions for use in the generating of the driving signal.

The aerosol-generating device may be a personal vaporiser for delivering nicotine or cannabinoids to a user. The aerosol-generating device may be a smoking device configured for non-thermally generating an inhalable aerosol.

The aerosol-generating device may comprise a heater to heat the aerosol-forming substrate, either before it is aerosolised by the aerosol-generating element or after it has formed an aerosol. Warming the aerosol-forming substrate may provide a more desirable user experience.

The aerosol-generating device may comprise multiple component parts. For example, the aerosol-generating device may comprise a main unit and cartridge. The cartridge may contain the reservoir of liquid aerosol-forming substrate. The main unit may comprise a power source and the control circuitry. The pump is advantageously contained within the main unit, but may be provided in the cartridge. The aerosol-generating element may be part of the main unit or part of the cartridge. The aerosol-generating device may comprise more than two parts. For example, the pump and aerosol-generating element may be contained in an atomising component that is detachable from a main unit containing the power source and a separate cartridge containing the liquid reservoir may attach to the atomising component.

In another aspect of the disclosure, there is provided a method of operating an aerosol-generating device. The aerosol-generating device comprises a liquid reservoir, an aerosol generating element configured to generate an aerosol from a liquid aerosol-forming substrate, the aerosol generating element comprising an electrically operated, vibrating transducer, and an electrically operated pump configured to deliver liquid from the liquid reservoir to the aerosol generating element. The method comprises controlling the operation of the pump in dependence on one or more operating parameters of the transducer.

This method allows the delivery of liquid to the aerosol generating element to be matched to the operating parameters of the transducer. So, if the transducer is operating at a higher frequency or greater power, the pump can be controlled to deliver more liquid to the pump. This may prevent dry conditions at the transducer. Similarly, if the transducer operates at a lower frequency or lower power level, the pump may be control to deliver less liquid. This may prevent or reduce overloading of the transducer with liquid.

The step of controlling may comprise controlling a rate of liquid delivery from the reservoir to the aerosol-generating element in dependence on one or more operating parameters of the transducer.

The one or more operating parameters may comprise one or more of: a drive voltage, a drive current, a resonant frequency, an impedance, a phase difference, a drive frequency a rate of change of impedance, a rate of change of current and a rate of change of voltage.

The method may further comprise: receiving a feedback signal from the transducer. The step of controlling may then comprise controlling the operation of the pump in dependence on the feedback signal.

The feedback signal may comprise one or more of: a resonant frequency, an impedance, and a phase. The feedback signal may comprise an amplitude or a rate of change of impedance, current or phase.

The method may comprise periodically controlling the operation of the pump in dependence on the feedback signal.

The method may comprise comparing the feedback signal with one or more thresholds to provide a comparison result and adjusting the operation of the pump in dependence on the comparison result.

The method may comprise adjusting the operation of the pump so as to maintain the feedback signal at a desired value or within a desired range of values. The method may comprise controlling the transducer based on the feedback signal.

The aerosol-generating device may comprise one or more sensors, and the method may comprise controlling the operating parameters of the transducer dependent on an output from one or more sensors.

The one or more sensors may comprise an airflow sensor. The method may comprise operating the pump in response to an output from the airflow sensor.

The one or more sensors may comprise a temperature sensor or a humidity sensor.

The one or more sensors may comprise a sensor for determining an identity of the liquid aerosol-forming substrate.

The method may comprise stopping operation of the pump a predetermined time before stopping operation of the transducer.

The aerosol-generating device may comprise a user interface. The method may comprise controlling the operating parameters of the transducer dependent on an input signal from the user interface.

The aerosol-generating device may comprise a memory. The method may comprise controlling the operating parameters of the transducer dependent on user preferences or pre-set values stored in memory.

The method may comprise controlling the transducer to operate in response to a user demand for aerosol.

The aerosol-generating device may be as described with reference to the first aspect of the disclosure. In particular, the aerosol-generating device used in the method of the second aspect of the disclosure may comprise any of the features of the aerosol-generating device described in relation to the first aspect of the disclosure.

In both aspects of the disclosure, the liquid aerosol-forming substrate employed may take many different forms. The following paragraphs describe various exemplary but nonlimiting materials and compositions for the liquid aerosol-forming substrate.

The liquid aerosol-forming substrate may comprise nicotine. The nicotine-containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise at least one aerosol-former. An aerosol-former is any suitable known compound or mixture of compounds that, in use, facilitates formation of a dense and stable aerosol. Suitable aerosol-formers are well known in the art and include, but are not limited to: polyhydric alcohols, such as triethylene glycol, 1,3-butanediol and glycerine; esters of polyhydric alcohols, such as glycerol mono , di-, or triacetate; and aliphatic esters of mono-, di-, or polycarboxylic acids, such as dimethyl dodecanedioate and dimethyl tetradecanedioate. Aerosol formers may be polyhydric alcohols or mixtures thereof, such as triethylene glycol, 1,3-butanediol and glycerine. The liquid aerosol-forming substrate may comprise other additives and ingredients, such as flavourants.

The liquid aerosol-forming substrate may comprise water.

The liquid aerosol-forming substrate may comprise nicotine and at least one aerosol former. The aerosol former may comprise glycerine. The aerosol-former may comprise propylene glycol. The aerosol former may comprise both glycerine and propylene glycol. The liquid aerosol-forming substrate may have a nicotine concentration of between about 2% and about 10%.

The first and second aspect of the disclosure relate to control of a pump based on one or more operating parameters of a transducer in an aerosol generating system. It is also possible to feed liquid to an aerosol-generating element passively, using gravity or a wicking material for example, instead of using a pump. In that case, the one or more operating parameters of the transducer may be used to control the transducer. The transducer feedback control can be used to adapt the operation of the transducer to the liquid load.

According to another aspect of the disclosure, there is provided an aerosol generating device. The aerosol-generating device may comprise a transducer. The transducer may be a piezoelectric transducer. The aerosol generating device may comprise drive circuitry connected to the transducer and configured to apply an oscillating current to the transducer. The aerosol-generating device may comprise control circuitry connected to the drive circuitry and configured to monitor one or more operating parameters of the transducer, the control circuitry configured to control the operation of the drive circuitry based on the one or more operating parameters of the transducer.

Examples will now be further described with reference to the figures, in which:
Figure 1 is a schematic of an aerosol-generating device in accordance with the disclosure;
Figure 2 illustrates delivery of liquid to a vibrating plate transducer;
Figure 3 is a schematic illustration of feedback control used in an embodiment of the disclosure;
Figure 4 is a schematic illustration of feedback control used in an embodiment of the disclosure;
Figures 5a and 5b illustrate the behaviour of a transducer under different liquid loads;
Figure 6 illustrates a measurement circuit for providing a measure of impedance and phase of a transducer for feedback;
Figure 7 illustrates feedback signals based on impedance and current; and
Figure 8 illustrates the behaviour of a transducer over time under variable liquid load.

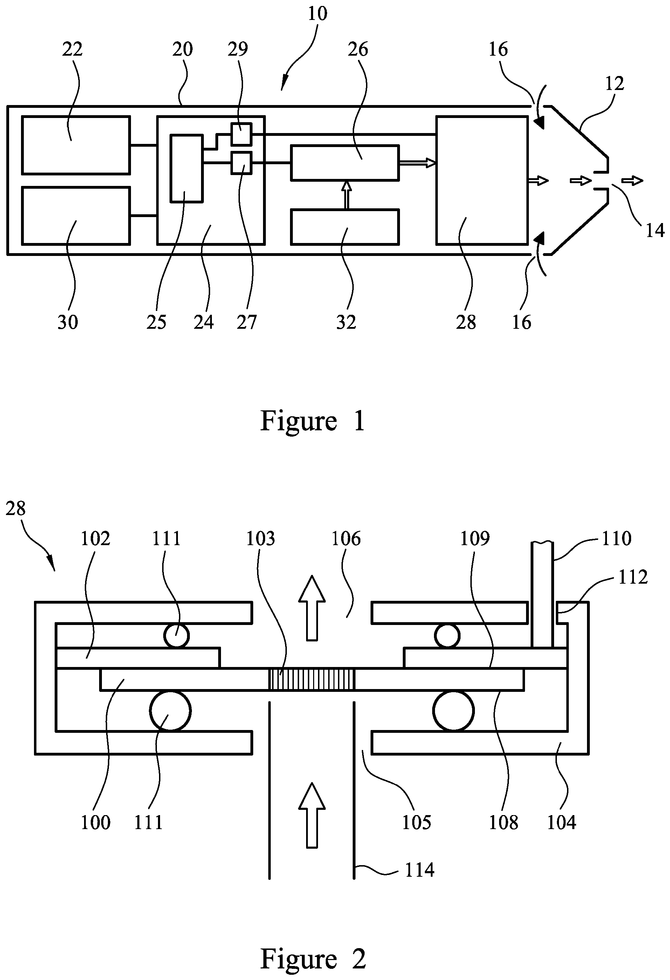

Figure 1 is a schematic of an aerosol-generating device comprising a pump and an aerosol-generating element. The aerosol-generating device 10 is a handheld vaporiser with a mouthpiece 12 through which generated aerosol can be inhaled by a user. The device comprises a housing 20 containing a power source 22 and control unit 24, a micropump 26, an transducer assembly 28 including an aerosol-generating element and a vibrating transducer, and user interface components 30 including an airflow sensor and at least one input button. A cartridge 32 containing a liquid aerosol-forming substrate, is received in the device, from which the micropump can deliver liquid aerosol-forming substrate to the transducer assembly 28.

The power source 22 is a rechargeable lithium ion battery. The battery is connected to the control unit 24 and provides power to both the micropump 26 and the transducer assembly 28 through the control unit. The control unit 24 comprises control circuitry 25, a pump drive circuit 27 and a transducer drive circuit 29. The control circuitry 25 may include a memory and a controller, for example, a field-programmable gate array (FPGA) or a programmable microcontroller (MCU). The characteristics of the vibrating transducer and micropump are stored in the memory and read out during operation.

The micropump may be may be a mechanical pump, a diaphragm pump, a piezoelectric pump, a peristaltic pump, an electro-hydrodynamic or magneto-hydrodynamic pump, an electro-osmotic pump, an adhesion pump, or rotary pump. An example of a suitable micropump is the mp6 micropump from Bartels Mikrotechnik GmbH, Konrad-Adenauer-Allee 11, 44263 Dortmund, Germany, which uses a piezoelectric diaphragm in combination with passive check valves.

The aerosol-generating element comprises a piezoelectric transducer that drives a perforated plate. Figure 2 illustrates the arrangement of the transducer and plate. As shown in Figure 2, the transducer assembly 28 comprises a vibratable plate 100 and transducer 102 housed inside an aerosol generator housing 104. The housing 104 comprises a hollow cylindrical box, having an inlet opening 105 and an outlet opening 106 arranged in co-axial alignment at opposite sides of the housing 104.

The vibratable plate 100 comprises a substantially circular aluminium disc, having a thickness of about 2 mm and a diameter of about 15 mm. A plurality of perforations 103 extends from an inlet side 108 to an opposing outlet side 109 of the vibratable plate. The plurality of perforations form an array having a substantially circular shape. The substantially circular array has a diameter of about 7 mm, and is arranged substantially centrally in the plate 100.

The perforations have a substantially circular cross-section and are tapered from the inlet side 108 to the outlet side 109 of the vibratable plate 100. The passages have a diameter at the inlet side of about 8 µm and a diameter at the outlet side of about 6 µm. The perforations are typically formed by high-speed laser drilling. The plurality of perforations is comprised of about 4000 perforations arranged with equal spacing across the array.

Transducer 102 comprises a piezoelectric transducer. The piezoelectric transducer is a substantially circular annular disc of piezoelectric material, typically zirconate titanate (PZT). The piezoelectric transducer has a thickness of about 2 mm, an outer diameter of about 17 mm and an inner diameter of about 8 mm.

As shown in Figure 2, the transducer 102 is in direct contact with the vibratable plate100, at the outlet side 109 of the vibratable plate. The inner diameter of the piezoelectric transducer 102 circumscribes the array of perforations 103, such that the open ends of the perforations at the outlet side are not covered by the piezoelectric transducer 102. In other embodiments (not shown) it is envisaged that the piezoelectric transducer 102 may be in direct contact with the vibratable plate 100 at the inlet side 108.

The vibratable plate 100 and piezoelectric transducer 102 are supported within the housing 104 by a pair of elastomeric O-rings 111, which allow the vibratable plate 100 and the piezoelectric transducer 102 to vibrate within the housing 104. The vibratable plate 100 and piezoelectric transducer 102 are held together by pressure from the opposing O-rings 111. However, in other embodiments (not shown) the vibratable plate 100 and the piezoelectric transducer 102 may be bonded by any suitable means, such as an adhesive layer.

The vibratable plate 100 and the piezoelectric transducer 102 are arranged within the housing 104 such that the array of perforations 103 is in coaxial alignment with the inlet and outlet openings 105, 106 of the housing 104.

One or more spring pins 110 extend through an opening 112 in the housing 104 to provide electrical connection of the piezoelectric transducer 102 to the control circuitry and the battery of the device. The one or more spring pins 110 are held in contact with the piezoelectric transducer 102 by pressure, rather than by a mechanical connection so that good electrical contact is maintained during vibration of the piezoelectric transducer 102.

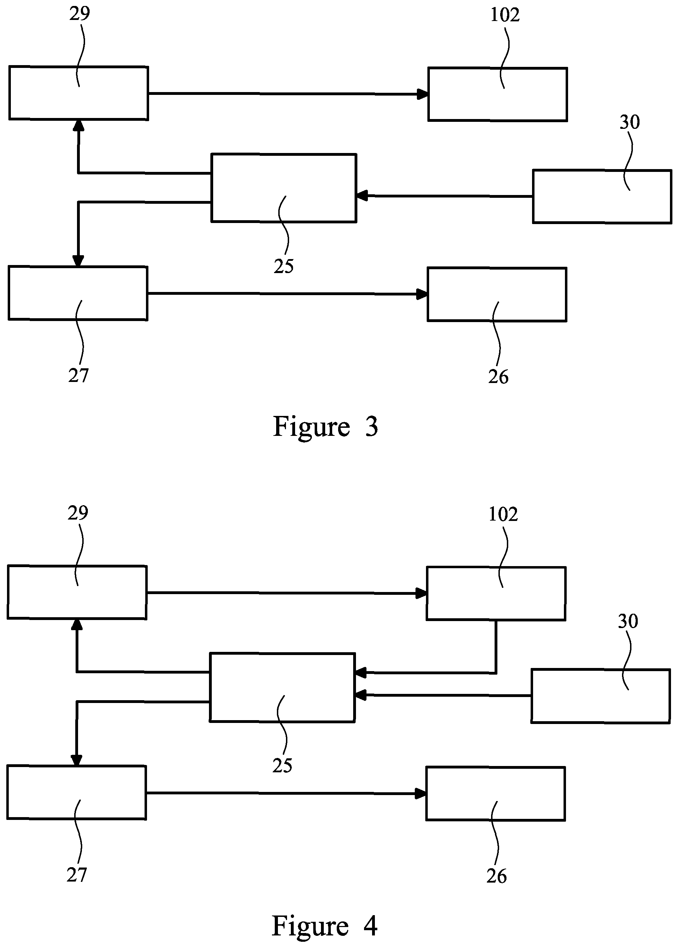

In use, the micropump 26 delivers liquid from the liquid reservoir to the perforated plate 100. As shown in Figure 3, a liquid delivery tube 114 extends into the housing 104 and ends adjacent to the inlet side 108 of the plate 100 at the array of perforations 103. In use, liquid aerosol-forming substrate (not shown) is delivered by the pump through the liquid delivery tube 114.

The device includes at least one air inlet 16 in the housing, an air outlet 14 through the mouthpiece and an airflow path that extends from the air inlet 14 to the air outlet 16 past the aerosol generating element. When a user draws on the mouthpiece air is drawn through the air inlet, past the vibratable plate 100 to the air outlet.

The user interface components 30 include an airflow sensor. The airflow sensor may be any suitable airflow sensor, such as a microphone. When a user draws on the air outlet of the mouthpiece, ambient air is drawn through air inlet. The airflow sensor is positioned to detect this airflow. When an airflow indicative of a user puff is detected by the airflow sensor, the control unit 24 activates the piezoelectric transducer 102 and the micropump 26. The battery supplies electrical energy to the piezoelectric transducer 102, under the control of the control unit 24, which vibrates, deforming in the thickness direction. The piezoelectric transducer 102 transmits the vibrations to the vibratable plate 100, which vibrates, also deforming in the thickness direction. The vibrations in the vibratable plate 100 deform the plurality of perforations 103, which draws liquid aerosol-forming substrate from the delivery tube114, through the plurality of perforations 103 at the inlet side 108 of the vibratable plate 100, and ejects atomised droplets of liquid aerosol-forming substrate from the passages at the outlet side 109 of the vibratable plate 100, forming an aerosol. The aerosol droplets ejected from the vibratable plate 100 mix with and are carried in the air flow from the inlet and are carried towards the air outlet 124 for inhalation by the user.

The user interface components may comprise an on/off button, so that the device must be switched on before the power can be supplied to the micropump or transducer. The user interface components may also include an interface that allows a user to select different operating modes or settings.

The control of the micropump and transducer is co-ordinated by the control unit. A first embodiment of a control scheme is shown in Figure 3. Figure 3 illustrates the control circuitry 25 connected to both a pump drive circuit 27 and a transducer drive circuit 29. The pump drive circuit 27 is connected to the pump 26 and the transducer drive circuitry 29 is connected to the transducer 102. In this embodiment the operating parameters of the transducer 102 are determined based on input from the airflow sensor 30 and on information stored in memory. The drive current supplied to the transducer has an initial frequency and wave shape based on parameters stored in memory. During manufacture of the device the frequency response of the transducer assembly, including the vibratable plate 100, can be characterised and an initial frequency and waveform set. The control circuitry 25 controls the transducer drive circuit to control the current supplied to the transducer and the pump drive circuitry to control the current supplied to the micropump. The drive current supplied to the transducer can then be modified based on the strength of the detected airflow. A higher detected airflow rate may give rise to a higher frequency or higher amplitude current being supplied to the transducer from the transducer drive circuit 29 in order to generate a greater volume of aerosol. The control circuitry 25 controls the pump drive circuit based on the parameters being applied to the transducer drive circuit. If a greater current is being supplied to the transducer to generate a particular throughput of aerosol, the corresponding pump parameters may be read from memory and the pump drive circuit controlled accordingly to ensure a matching amount of liquid is delivered to the aerosol generating element.

In another embodiment the control unit may receive a signal feedback from the transducer indicating the condition of the transducer, for example whether there is enough, not enough or too much liquid on the transducer. This arrangement is illustrated in Figure 4. Figure 4 again illustrates the control circuitry 25 connected to both a pump drive circuit 27 and a transducer drive circuit 29. The pump drive circuit 27 is connected to the pump 26 and the transducer drive circuitry 29 is connected to the transducer 102. However, in the arrangement of Figure 4 a feedback signal from the transducer is provided to the control circuitry. The feedback signal indicates the state of the load on the transducer, and may be, for example, resonance frequency, impedance or phase. The load on the transducer depends on how much liquid is present at the perforated plate 100. So the feedback signal is indicative of whether there is enough, not enough or too much liquid at the perforated plate.

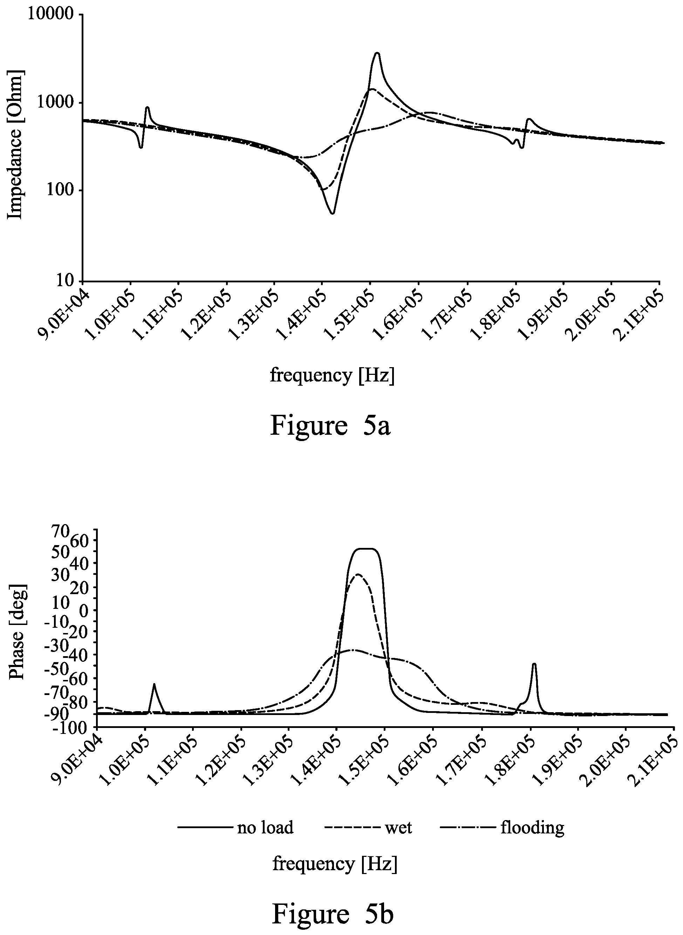

Figures 5a and 5b illustrate the behaviour of a transducer under different liquid loads that can form the feedback signal. Figure 5a shows the impedance over a frequency range covering the transducer's main resonance peak. The solid line shows the impedance with no load on the transducer, the dashed line shows impedance for liquid load under normal operating conditions to generate aerosol, and the dash-dotted line shows the impedance for too much liquid on the perforated plate, which might occur if the pump is supplying too much liquid. It can be seen that the pump impedance can be used as an indicator of liquid conditions at the perforated plate. Figure 5b shows the phase of the transducer over the same frequency range. Again the solid line shows the phase with no liquid on the transducer, the dashed line shows phase for liquid load under normal operating conditions to generate aerosol, and the dash-dotted line shows the phase for too much liquid on the perforated plate. For a fixed operating frequency, the phase angle decreases with increasing liquid load and may turn the reactance of the transducer capacitive. Increasing liquid load also lowers the resonance frequency of the transducer.

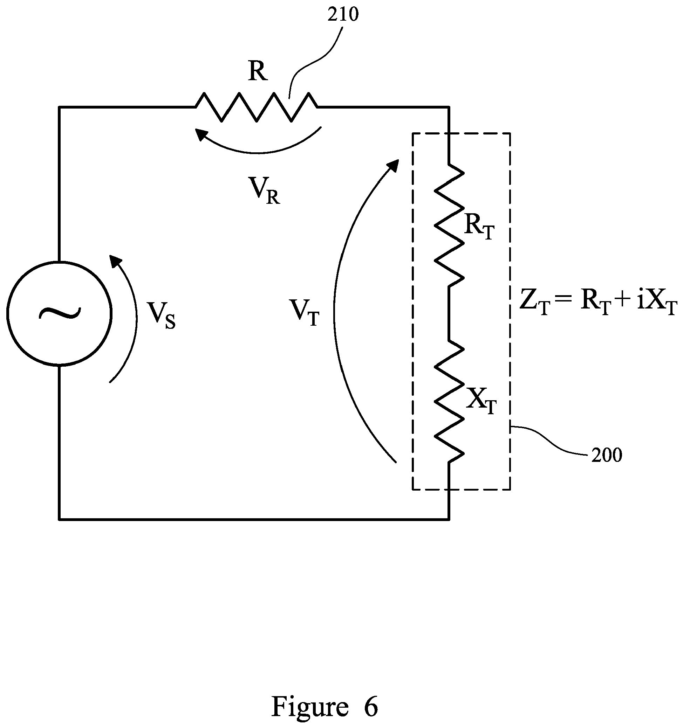

Providing a feedback signal indicative of the load on the perforated plate allows the control circuitry to adjust the control of the pump to avoid overloading or under loading. The feedback signal can be provided to the control circuitry continuously. There are various methods that can be employed for measurement of impedance, phase and frequency. For example, bridge circuits, such as a Wheatstone bridge, integrated circuits such as AD5941 and CD4040, phase comparator or phase frequency detectors based on logic gates, or an SoC, for example Xilinx Zynq 710. Figure 6 illustrates a measurement circuit for providing a measure of impedance and phase of a transducer for feedback. The transducer is illustrated by dashed box 200 as comprising a resistance and R_{T} and a reactance X_{T}. A series resistor 210 is used to measure the current flow into the transducer. A voltage V_{S} having a fixed frequency and amplitude is applied by the transducer drive circuit and the voltage across the transducer V_{T} and across the resistor V_{R} are measured. This allows for the calculation of the impedance Z_{T} of the transducer and the phase θ= arc tan (X_{T}/R_{T}).

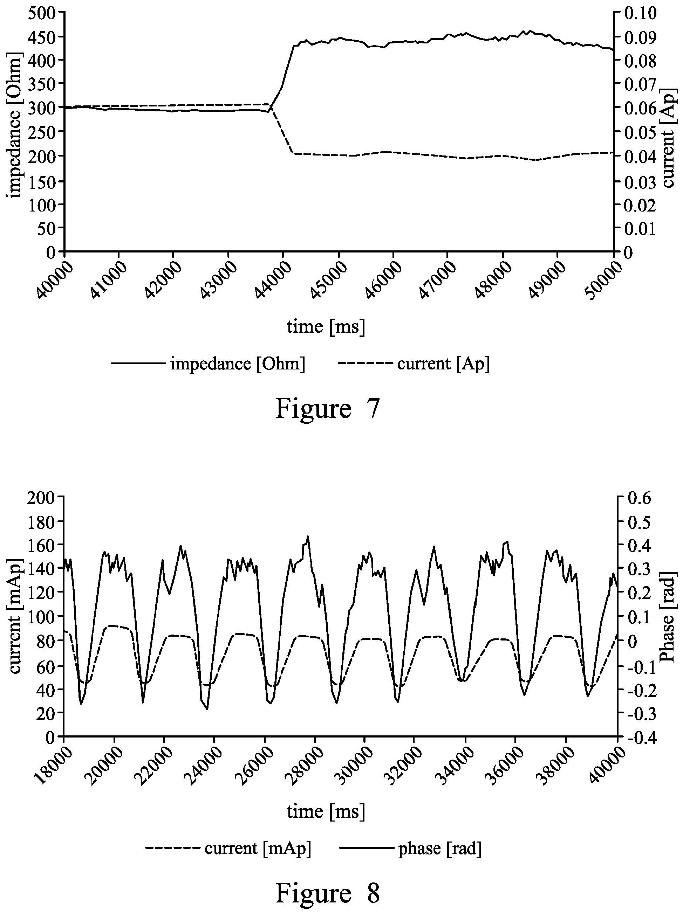

The control circuitry uses the feedback signal to alter the current or voltage applied to the pump by the pump drive circuit. Figure 7 illustrates feedback signals based on impedance and current. The impedance over time is shown as the solid line. The corresponding current is shown as a dotted line. The current under stable load conditions, on the right side of Figure 7 is around 40mA. Current increases as load decreases. So the control circuitry may be configured to detect when a threshold of current is reaches, say 50mA, indicating too little liquid is being supplied. So, as shown in Figure 7, initially there is too little liquid being delivered and the current is above the 50mA threshold. The control circuitry increases the supply of liquid from the pump until the stable conditions are reached. The monitoring of the feedback signal and the comparison to a threshold or thresholds can be carried out on a continuous basis during operation of the transducer.

A similar control can be based on impedance as illustrated by Figure 7, with impedance thresholds used in the same way as current thresholds. In the example shown in Figure 7, the load impedance in stable conditions is in the range of 420 Ohms to 470 Ohms. Whenever the impedance is outside of a window, for example falling below 400 Ohms or above 500 Ohms, the control circuitry sends modified driving parameters to the pump drive circuit to maintain stable aerosol generation, or alternatively to stop aerosol generation.

If the control circuitry stops aerosol generation based on the feedback signal, the pump drive signal is stopped first. After a predetermined time period, the transducer drive signal is stopped. This ensures that liquid already at the transducer is turned into aerosol and is not replaced with more liquid. This reduces the possibility of liquid leaking from the device after the device has stopped operating.

As an alternative to using fixed thresholds, the rate of change of impedance, current or phase can be monitored by the control circuitry to determine how to modify the pump drive signal or the transducer drive signal. Changes in impedance greater than a predetermined amount in a predetermined time period can indicate unstable conditions at the aerosol generating element.

Figure 8 illustrates how phase provides a feedback signal indicative of the load on the transducer over time. Figure 8 shows the phase and current over time of a transducer operated at a fixed frequency and voltage. The supply of liquid to the transducer from the pump is set too low for the rate of aerosol generation by the transducer. The mismatch between the transducer throughput and the pump flow rate leads to discontinuous aerosol generation. The liquid applied to the transducer is quickly turned into aerosol and there is then a delay before sufficient liquid is delivered. While the transducer is unloaded the phase angle is large and positive. When liquid is added, the phase angle steeply drops. In this example, the transducer takes only around 0.4 seconds to become unloaded again. Using phase angle, the control circuitry can adjust the operation of the pump to deliver liquid at a higher rate, providing steady state conditions at the transducer. Using phase as a feedback signal has an advantage that it is not significantly affected by changes in the magnitude of the voltage applied to the transducer.

The feedback signal may also be used to determine the drive signal provided to the transducer. For example, if there is decrease in liquid load detected the drive signal to the transducer can be altered to reduce throughput and balance throughput with the delivery rate of the pump. The system is able to both provide a variable rate of aerosol generation to meet a user's needs and stable load conditions at the aerosol generating element.

It may also be useful to tune the driving frequency of the transducer based on the feedback signal in order optimise efficiency. If the transducer impedance is matched to the impedance of the transducer drive circuit power efficiency can be maximised. As the impedance of the transducer changes with changing load conditions, the feedback signal can be used to modify the driving frequency of the transducer so as to maintain the same impedance.

## Claims

1. An aerosol-generating device (10) comprising:
a reservoir containing a liquid aerosol-forming substrate;
an aerosol-generating element configured to generate an aerosol from the liquid aerosol-forming substrate, the aerosol-generating element comprising an electrically operated, vibrating transducer (102);
an electrically operated pump (26) configured to deliver liquid aerosol-forming substrate from the reservoir to the aerosol-generating element; and
control circuitry (25) connected to the pump (26) and the transducer (102), the control circuitry (25) configured to control the operation of the pump (26) in dependence on one or more operating parameters of the transducer (102).

2. An aerosol-generating device (10) according to claim 1, wherein the control circuitry (25) is configured to control a rate of delivery of liquid from the reservoir to the aerosol-generating element in dependence on one or more operating parameters of the transducer (102).

3. An aerosol-generating device (10) according to claim 1 or claim 2, wherein the control circuitry (25) is configured to drive the transducer (102).

4. An aerosol-generating device (10) according to any preceding claim, wherein the one or more operating parameters comprises one or more of: a drive voltage, a drive current, a resonant frequency, an impedance, a phase difference, a drive frequency a rate of change of impedance, a rate of change of current and a rate of change of voltage.

5. An aerosol-generating device (10) according to any preceding claim, wherein the control circuitry (25) is configured to receive a feedback signal from the transducer (102) and is configured to control the operation of the pump (26) in dependence on the feedback signal.

6. An aerosol-generating device (10) according to claim 5, wherein the feedback signal comprises one or more of: a resonant frequency, an impedance, and a phase.

7. An aerosol-generating device (10) according to claim 5 or 6, wherein the control circuitry (25) is configured to periodically control the operation of the pump (26) in dependence on the feedback signal.

8. An aerosol-generating device (10) according to claim 5, 6 or 7, wherein the control circuitry (25) is configured to compare the feedback signal with one or more thresholds to provide a comparison result and to alter operation of the pump (26) in dependence on the comparison result.

9. An aerosol-generating device (10) according to claim 8, wherein the control circuitry (25) is configured to alter the operation of the pump (26) if the feedback signal is not a desired value or within a desired range of values.

10. An aerosol-generating device (10) according to any one of claims 5 to 9, wherein the control circuitry (25) is configured to control the transducer (102) based on the feedback signal.

11. An aerosol-generating device (10) according to any preceding claim, wherein the aerosol-generating element can be controlled by the control circuitry (25) to operate in different modes of vibration.

12. An aerosol-generating device (10) according to any preceding claim, further comprising a sensor connected to the control circuitry (25), wherein the control circuitry (25) is configured to control the operating parameters of the transducer dependent on an output from one or more sensors.

13. An aerosol-generating device (10) according to any preceding claim, comprising a mouthpiece (12) through which a user can draw generated aerosol.

14. A method of operating an aerosol-generating device (10), the aerosol-generating device (10) comprising a liquid reservoir, an aerosol generating element configured to generate an aerosol from a liquid aerosol-forming substrate, the aerosol generating element comprising an electrically operated, vibrating transducer (102), and an electrically operated pump (26) configured to deliver liquid from the liquid reservoir to the aerosol generating element, the method comprising:
controlling the operation of the pump (26) in dependence on one or more operating parameters of the transducer (102).

15. A method according to claim 14, wherein the step of controlling comprises controlling a rate of liquid delivery from the reservoir to the aerosol-generating element in dependence on one or more operating parameters of the transducer (102).

## Patentansprüche

1. Aerosolerzeugungsvorrichtung (10), aufweisend:
einen Vorratsbehälter, der ein flüssiges aerosolbildendes Substrat enthält;
ein aerosolerzeugendes Element, das dazu eingerichtet ist, ein Aerosol aus dem flüssigen aerosolbildenden Substrat zu erzeugen, das aerosolerzeugende Element aufweisend einen elektrisch betriebenen, vibrierenden Wandler (102);
eine elektrisch betriebene Pumpe (26), die dazu eingerichtet ist, flüssiges aerosolbildendes Substrat aus dem Vorratsbehälter zu dem aerosolerzeugenden Element zu fördern; und
eine Regelschaltung (25), die mit der Pumpe (26) und dem Wandler (102) verbunden ist, die Regelschaltung (25) dazu eingerichtet ist, den Betrieb der Pumpe (26) in Abhängigkeit von einem oder mehreren Betriebsparametern des Wandlers (102) zu regeln.

2. Aerosolerzeugungsvorrichtung (10) nach Anspruch 1, wobei die Regelschaltung (25) dazu eingerichtet ist, eine Geschwindigkeit der Abgabe von Flüssigkeit aus dem Vorratsbehälter zu dem aerosolerzeugenden Element in Abhängigkeit von einem oder mehreren Betriebsparametern des Wandlers (102) zu regeln.

3. Aerosolerzeugungsvorrichtung (10) nach Anspruch 1 oder Anspruch 2, wobei die Regelschaltung (25) dazu eingerichtet ist, den Wandler (102) anzusteuern.

4. Aerosolerzeugungsvorrichtung (10) nach einem beliebigen vorhergehenden Anspruch, wobei der eine oder die mehreren Betriebsparameter einen oder mehrere umfassen von: eine Ansteuerspannung, einen Ansteuerstrom, eine Resonanzfrequenz, eine Impedanz, eine Phasendifferenz, eine Ansteuerfrequenz, eine Änderungsgeschwindigkeit der Impedanz, eine Änderungsgeschwindigkeit des Stroms und eine Änderungsgeschwindigkeit der Spannung.

5. Aerosolerzeugungsvorrichtung (10) nach einem beliebigen vorhergehenden Anspruch, wobei die Regelschaltung (25) dazu eingerichtet ist, ein Rückkopplungssignal von dem Wandler (102) zu empfangen, und dazu eingerichtet ist, den Betrieb der Pumpe (26) in Abhängigkeit von dem Rückkopplungssignal zu regeln.

6. Aerosolerzeugungsvorrichtung (10) nach Anspruch 5, wobei das Rückkopplungssignal eines oder mehrere umfasst von: einer Resonanzfrequenz, einer Impedanz, und einer Phase.

7. Aerosolerzeugungsvorrichtung (10) nach Anspruch 5 oder 6, wobei die Regelschaltung (25) dazu eingerichtet ist, den Betrieb der Pumpe (26) in Abhängigkeit von dem Rückkopplungssignal regelmäßig zu regeln.

8. Aerosolerzeugungsvorrichtung (10) nach Anspruch 5, 6 oder 7, wobei die Regelschaltung (25) dazu eingerichtet ist, das Rückkopplungssignal mit einem oder mehreren Schwellenwerten zu vergleichen, um ein Vergleichsergebnis vorzusehen und den Betrieb der Pumpe (26) in Abhängigkeit von dem Vergleichsergebnis zu ändern.

9. Aerosolerzeugungsvorrichtung (10) nach Anspruch 8, wobei die Regelschaltung (25) dazu eingerichtet ist, den Betrieb der Pumpe (26) zu ändern, wenn das Rückkopplungssignal kein gewünschter Wert ist oder nicht innerhalb eines gewünschten Bereichs von Werten liegt.

10. Aerosolerzeugungsvorrichtung (10) nach einem der Ansprüche 5 bis 9, wobei die Regelschaltung (25) dazu eingerichtet ist, den Wandler (102) basierend auf dem Rückkopplungssignal zu regeln.

11. Aerosolerzeugungsvorrichtung (10) nach einem beliebigen vorhergehenden Anspruch, wobei das aerosolerzeugende Element durch die Regelschaltung (25) regelbar ist, um in verschiedenen Vibrationsmodi zu arbeiten.

12. Aerosolerzeugungsvorrichtung (10) nach einem beliebigen vorhergehenden Anspruch, ferner aufweisend einen mit der Regelschaltung (25) verbundenen Sensor, wobei die Regelschaltung (25) dazu eingerichtet ist, die Betriebsparameter des Wandlers in Abhängigkeit von einer Ausgabe von einem oder mehreren Sensoren zu regeln.

13. Aerosolerzeugungsvorrichtung (10) nach einem beliebigen vorhergehenden Anspruch, aufweisend ein Mundstück (12), durch das ein Benutzer erzeugtes Aerosol ziehen kann.

14. Verfahren für ein Betreiben einer Aerosolerzeugungsvorrichtung (10), die Aerosolerzeugungsvorrichtung (10) aufweisend einen Flüssigkeitsvorratsbehälter, ein aerosolerzeugendes Element, das dazu eingerichtet ist, ein Aerosol aus einem flüssigen aerosolbildenden Substrat zu erzeugen, das aerosolerzeugende Element aufweisend einen elektrisch betriebenen, vibrierenden Wandler (102) und eine elektrisch betriebene Pumpe (26), die dazu eingerichtet ist, Flüssigkeit aus dem Flüssigkeitsvorratsbehälter an das aerosolerzeugende Element zu fördern, das Verfahren umfassend:
Regeln des Betriebs der Pumpe (26) in Abhängigkeit von einem oder mehreren Betriebsparametern des Wandlers (102).

15. Verfahren nach Anspruch 14, wobei der Schritt des Regelns ein Regeln einer Geschwindigkeit der Flüssigkeitsabgabe aus dem Vorratsbehälter zu dem aerosolerzeugenden Element in Abhängigkeit von einem oder mehreren Betriebsparametern des Wandlers (102) umfasst.

## Revendications

1. Dispositif de génération d'aérosol (10) comprenant :
un réservoir contenant un substrat formant aérosol liquide ;
un élément de génération d'aérosol configuré pour générer un aérosol à partir du substrat formant aérosol liquide, l'élément de génération d'aérosol comprenant un transducteur vibrant à fonctionnement électrique (102) ;
une pompe à fonctionnement électrique (26) configurée pour distribuer un substrat formant aérosol liquide du réservoir à l'élément de génération d'aérosol ; et
une circuiterie de commande (25) raccordé à la pompe (26) et au transducteur (102), la circuiterie de commande (25) étant configurée pour commander le fonctionnement de la pompe (26) en fonction d'un ou de plusieurs paramètres de fonctionnement du transducteur (102).

2. Dispositif de génération d'aérosol (10) selon la revendication 1, dans lequel la circuiterie de commande (25) est configurée pour commander un taux de distribution de liquide du réservoir à l'élément de génération d'aérosol en fonction d'un ou plusieurs paramètres de fonctionnement du transducteur (102).

3. Dispositif de génération d'aérosol (10) selon la revendication 1 ou la revendication 2, dans lequel la circuiterie de commande (25) est configurée pour attaquer le transducteur (102).

4. Dispositif de génération d'aérosol (10) selon l'une quelconque des revendications précédentes, dans lequel les un ou plusieurs paramètres de fonctionnement comprennent un ou plusieurs parmi : une tension d'attaque, un courant d'attaque, une fréquence de résonance, une impédance, une différence de phase, une fréquence d'attaque, un taux de variation d'impédance, un taux de variation de courant et un taux de variation de tension.

5. Dispositif de génération d'aérosol (10) selon l'une quelconque des revendications précédentes, dans lequel la circuiterie de commande (25) est configurée pour recevoir un signal de rétroaction provenant du transducteur (102) et est configuré pour commander le fonctionnement de la pompe (26) en fonction du signal de rétroaction.

6. Dispositif de génération d'aérosol (10) selon la revendication 5, dans lequel le signal de rétroaction comprend une ou plusieurs parmi : une fréquence de résonance, une impédance et une phase.

7. Dispositif de génération d'aérosol (10) selon la revendication 5 ou 6, dans lequel la circuiterie de commande (25) est configurée pour commander périodiquement le fonctionnement de la pompe (26) en fonction du signal de rétroaction.

8. Dispositif de génération d'aérosol (10) selon la revendication 5, 6 ou 7, dans lequel la circuiterie de commande (25) est configurée pour comparer le signal de rétroaction à un ou plusieurs seuils pour fournir un résultat de comparaison et pour modifier le fonctionnement de la pompe (26) en fonction du résultat de comparaison.

9. Dispositif de génération d'aérosol (10) selon la revendication 8, dans lequel la circuiterie de commande (25) est configurée pour modifier le fonctionnement de la pompe (26) si le signal de rétroaction n'est pas une valeur souhaitée ou au sein d'une plage de valeurs souhaitée.

10. Dispositif de génération d'aérosol (10) selon l'une quelconque des revendications 5 à 9, dans lequel la circuiterie de commande (25) est configurée pour commander le transducteur (102) sur la base du signal de rétroaction.

11. Dispositif de génération d'aérosol (10) selon l'une quelconque des revendications précédentes, dans lequel l'élément de génération d'aérosol peut être commandé par la circuiterie de commande (25) pour fonctionner dans différents modes de vibration.

12. Dispositif de génération d'aérosol (10) selon l'une quelconque des revendications précédentes, comprenant en outre un capteur raccordé à la circuiterie de commande (25), dans lequel la circuiterie de commande (25) est configurée pour commander les paramètres de fonctionnement du transducteur en fonction d'une sortie d'un ou plusieurs capteurs.

13. Dispositif de génération d'aérosol (10) selon l'une quelconque des revendications précédentes, comprenant un embout buccal (12) à travers lequel un utilisateur peut tiré l'aérosol généré.

14. Procédé de fonctionnement d'un dispositif de génération d'aérosol (10), le dispositif de génération d'aérosol (10) comprenant un réservoir de liquide, un élément de génération d'aérosol configuré pour générer un aérosol à partir d'un substrat formant aérosol liquide, l'élément de génération d'aérosol comprenant un transducteur vibrant à fonctionnement électrique (102) et une pompe à fonctionnement électrique (26) configurée pour distribuer du liquide provenant du réservoir de liquide à l'élément de génération d'aérosol, le procédé comprenant :
la commande du fonctionnement de la pompe (26) en fonction d'un ou de plusieurs paramètres de fonctionnement du transducteur (102).

15. Procédé selon la revendication 14, dans lequel l'étape de commande comprend la commande d'un taux de distribution de liquide du réservoir à l'élément de génération d'aérosol en fonction d'un ou plusieurs paramètres de fonctionnement du transducteur (102).
